# EUROPEAN PATENT APPLICATION

(11) **EP 1 820 439 A1**
(43) Date of publication of application: **22.08.2007**
(21) Application number: 07003482.2
(22) Date of filing: 20.02.2007
(51) Int. Cl.: A61B 1/04, A61B 1/267

(54) **Wireless optical endoscopic device**

(30) Priority: 21.02.2006 US 358201
(71) Applicant: Karl Storz Endovision, Charlton, MA 01507 (US)
(72) Inventor: Birnkrant, Dashiell, US-Worcester MA 01605 (US)
(74) Representative: Heuckeroth, Volker

(57) **Abstract**

A video endoscopy system (100) for displaying an area to be viewed to a user, the system providing for wireless transmission of image data representative of the area to be viewed. The video endoscopy system (100) uses an LED (108) and a battery (110) for providing illuminating light to the area to be viewed. The video endoscopy system (100) also uses a C-Mos chip (106) for picking up reflected light from the area to be viewed and generating image data representative of the reflected light, which in turn is wirelessly transmitted to a video system (112) for display to the user.

## Description

### Field Of The Invention

The invention relates to a video endoscopic device, and more particularly to a wireless transmitting endoscopic device for use in non-invasive surgical and intubation procedures.

### Background Of The Invention

In the United States, approximately 20 million patients are operated on and anesthetized each year. Approximately 50% of surgeries are performed using general anesthesia, which means the patient is put to sleep and the ventilation and other physiological functions are monitored. While anesthetized, the patient's breathing functions are temporarily disabled. Ventilation is therefore supplied to the patient by the anesthesiologist during the procedure.

Ventilation is provided through an endotracheal tube. This tube is inserted into the trachea, and it is closed against the wall of the trachea by an inflatable cuff. The insertion of this tube involves risks that the anesthesiologist seeks to avoid or at least minimize. It is estimated that between one in 6,000 to one in 8,000 general anesthesia procedures result in death. There are of course many causes but of these it is estimated that about one third of them are caused by the intubation procedure.

The foremost obstacles encountered by the anesthesiologist include; the remoteness of the location where the tube is to be positioned, the consequent restriction of view as the tube is inserted, variations and anomalies in the anatomy of the patients, an uncomfortable and unnatural position for the anesthesiologist while holding the instrument, the potential need to change blades during the procedure, and the necessity for rapid intubation.

It should be noted that when the tube is inserted, the patient is asleep hyperoxygenated and then paralyzed for the procedure, and therefore not breathing. In addition, the ventilator is not yet in operation. This gives the anesthesiologist only about two minutes in which to intubate the patient, inflate the cuff, and start ventilation. If he is delayed because of unsuccessful attempts, he must stop, apply a ventilation mask to the patient, supply oxygen for a time through the mask, remove the mask, adjust medication if necessary, and then start over again. This delays the operation and extends the patient's time under anesthesia. This extension of time while under anesthesia may have very serious consequences, especially for elderly patients.

With the advent of endoscopic equipment and small cameras, instrumentation has been improved to the extent that it can enable viewing of the cords and larynx on a video screen thereby facilitating the intubation of the patient in a relatively quick and safe manner. Systems typically use, for example a Charge-Coupled Device (CCD) as the image sensor, in the form of a light-sensitive chip that converts the optical signals into electrical signals that are conveyed from the CCD to, for example, an image-sensing camera module. However, such systems typically use an illumination source, which supplies illuminating light to the area ahead of the device via an illumination cable, and transmit images picked up by the CCD back to a video monitor via an image cable. The cabling and light guides can add complexity and to the system and increase the corresponding size and weight of the device.

Endoscopes are now widely used in minimally invasive surgery. Endoscopes typically contain a light guiding system, usually in the form of fiber optic cables, in order to bring light to the surgical area. The light guiding system typically extends through the handle of the laryngoscope and through a guide tube located in the blade so as to position the light guiding system to illuminate the area ahead of the blade. Endoscopes also typically contain an image guiding system, for example in the form of a rigid rod lens system, arranged in the shaft of the endoscope. The image guiding system can also be configured as an ordered, flexible fiber optic bundle. The image guiding system is utilized to transmit reflected light from the area ahead of the blade to a camera. The camera, attached at the proximal end of the endoscope, usually contains a CCD sensor. The image guide typically extends from the distal end of the device through the guide tube and then through for example, a handle of the device.

Typically, the combination light guiding system and image guiding system are permanently attached to the handle and are continuous, extending from the distal end of the device, through a handle and to the camera for the image guiding system, and to the light source for the light guiding system. Therefore, the light guiding system and image guiding system extending from the handle for insertion into the guide tube typically comprise flexible coherent fiber optic bundles. However, when reconfiguring the device, the bundle must be carefully inserted or withdrawn from the opening of the guide tube. This may take an unacceptable amount time for the physician to thread the bundle into the tube if the device must be reconfigured in the middle of the intubation process.

The light and image guiding systems have typically been permanently attached to the handle to ensure the system will reliably transmit the illuminating light and reflected images. To utilize a detachably connectable light and image guiding system, the attachment means has to rigidly hold the member in place such that the light and image guiding systems did not become misaligned. In addition, the attachment means must be easy and quick to operate, making it possible to perform the coupling procedure with as little close attention as possible, but nevertheless reliably.

In addition, the flexible bundles may easily be damaged and will wear over time, degrading or rendering the system inoperable. As a visual inspection of the device often will not indicate whether the bundles are damaged, it is conceivable that a physician may obtain a damaged or malfunctioning laryngoscope not realizing that it is damaged. The time involved with determining that the instrument is malfunctioning, withdrawing it, finding another laryngoscope, and then intubating the patient may have severe adverse effects upon the patient under anesthesia.

Further, laryngoscopes, as with most medical equipment, must be sterilized after use. Because the light and image guiding systems are permanently attached to the handle, they are exposed to extremely high temperatures, which also cause wear and/or failure of the flexible bundles. Also, because the light and image guiding systems are subjected to the sterilization process with the handle and blades, the handle must be hermetically sealed which may greatly add to the cost in manufacturing such a device.

### Summary Of The Invention

It is therefore desired to provide an improved video imaging system for use in an endoscopic device that reduces the complexity and size of present systems.

It is also desired to provide an improved video imaging system for use in an endoscopic device that reduces the time required for changing or reconfiguring the device.

It is further desired to provide an improved video imaging system for use in an endoscopic device that will achieve the above-listed benefits while still reducing the cost associated with the manufacture of the device.

It is still further desired to provide an improved video imaging system for use in a laryngoscope that minimizes the problems associated with having the guides extend from the end of the blade to the handle and from the handle to video equipment.

These and other objectives are achieved by providing an endoscopic device that utilizes a digital imaging chip located in the endoscopic device. In addition, a Light Emitting Diode (LED) may further be located in the endoscopic device for illumination of an area to be viewed.

It is contemplated that the digital imaging chip may comprise either a CCD or a C-Mos chip.

Further, it is contemplated that the digital imaging chip may be provided as a wireless device for wirelessly transmitting image data picked up from the area to be viewed. This provides a number of significant advantages. First, wireless transmission of data allows for both the light and the image guides to the device to be eliminated. For flexible endoscopes, this means that the costs associated with the provision of, for example, coherent fiber optical cables may be reduced. In addition, the wear and tear that such cables endure through normal use and manipulation is also avoided. Still further, the size of the device, i.e. the diameter, may be reduced because flexible portion no longer has to maintain light or image guides therein.

In the case of a video laryngoscope, the light and image guides, whether flexible cables or a rigid attachment member, may be eliminated. In this manner, a physician no longer has to attach or be concerned with the threading of cables into guides because the cables have been eliminated. This allows for a quicker change of blades and a faster intubation of the patient with, for example, a laryngoscope.

The elimination of light and image guides also allows design for the device, whether an endoscope or a laryngoscope, to be simpler and less cumbersome. Especially is this the case where the endoscope or laryngoscope is provided completely wireless, leaving the physician free to move and manipulate the device without regard to wires or cables.

For video endoscopes, the digital imaging chip may, in one advantageous embodiment, be positioned at the distal end of the flexible endoscope. An LED is positioned adjacent to the digital imaging chip may be provided with a battery that may last for example, up to for instance, 12 hours. Alternatively, it is contemplated that the LED and/or the digital imaging chip may individually or both, be located at a proximal end of the endoscope or in the endoscope handle. In the case where either the LED and/or the digital imaging chip are positioned at a proximal end of the endoscope or in the handle, it is contemplated that an illuminating light guide will be positioned within the flexible endoscope for transmitting illuminating light to the area to be viewed ahead of the endoscope. Likewise, when the digital imaging chip is located at a proximal end of the endoscope or in the handle, an image guide will need to be located within the flexible endoscope for transmitting reflected light back to the digital imaging chip.

Similar configurations may be used for video laryngoscope applications, the digital imaging chip may positioned at either the distal or proximal ends of the laryngoscope blade or in the handle along with the LED. It is contemplated that the blade or the handle may be provided with a cavity for receiving the digital imaging chip and LED, such that the video/illumination device is removable from the blade or the handle. In this manner the blade or the handle may be sterilized as normal and a single video/illumination device may be used with multiple blades. This would also allow for repair and/or replacement of the video/illumination device if it became damaged.

It is also contemplated that the image signal generated by the digital imaging chip may be wirelessly transmitted to a video system for display. The wireless transmission from the digital imaging chip allows for the benefits previously described herein. A memory unit may also be provided for recording of the procedure. The memory unit may be provided in, for example, the endoscopic device so that, in the event there is a communication lapse between the digital imaging chip and the video display, the gathered image data may be buffered to allow the physician to monitor the positioning of the device after any possible interruption.

It is further contemplated that a window covering a cavity may be provided such that, in one embodiment, the video/illumination module may be removably inserted into the cavity. Alternatively, the video/illumination module may be removably or permanently affixed to the handle.

The wireless transmission therefore, allows for a smaller sized device, a simpler design, no wires or cables to deal with allowing greater ease of movement for the physician, lower cost, and interchangeability.

Accordingly, in one advantageous embodiment of the present invention, a video endoscope system for displaying image data to a user is provided comprising an endoscopic device for coupling to a video system, the endoscopic device having a proximal end connected to a handle and a distal end and including, a video/illumination device associated with the endoscopic device, the video/illumination device having an illumination device and a battery for illuminating an area to be viewed, and a digital imaging chip for picking up reflected light from the area and generating image data. The system is provided such that the image data is wirelessly transmitted to the video system for display to a user.

In another advantageous embodiment, a video endoscope system for displaying image data to a user is provided comprising, a flexible endoscope for coupling to a video system, the flexible endoscope having a proximal end connected to a handle and a distal end and including, and a digital imaging chip and an illuminating device associated with the flexible endoscope, the illuminating device having a battery for illuminating an area to be viewed, and the digital imaging chip for picking up reflected light from the area and for generating image data. The system is provided such that the image data is wirelessly transmitted to the video system for display to a user.

In still another advantageous embodiment, a video laryngoscope system is provided for displaying image data to a user. The system comprises a video laryngoscope for coupling to a video system, the video laryngoscope having a blade with a proximal end connected to a handle and a distal end. The system further comprises a digital imaging chip and an illuminating device associated with the video laryngoscope, the illuminating device having a battery for illuminating an area to be viewed, and the digital imaging chip for picking up reflected light from the area and for generating image data. The system is provided such that the image data is wirelessly transmitted to the video system for display to a user.

In yet another advantageous embodiment, a method for viewing an area with an endoscopic device is provided comprising the steps of, positioning a digital imaging chip and an illuminating device on the endoscopic device, and wirelessly coupling the endoscopic device to a video system. The method further comprises the steps of, illuminating an area to be viewed with the illumination device and powered by a battery, and generating image data based on reflected light picked up by the digital imaging chip. The method still further comprises the steps of, wirelessly transmitting the image data to the video system, and displaying the image data to a user.

In still another advantageous embodiment, a video endoscope system for wirelessly transmitting and displaying image data to a user is provided comprising, an endoscopic device. The endoscopic device includes an illuminating device for illuminating an area to be viewed, a power source, coupled to and for powering the illuminating device, and a digital imaging chip for picking up reflected light from the area and for generating image data. The system is provided such that the digital imaging chip is wirelessly coupled to a video system via a coupling circuit for receiving the image data. The system is further provided such that the image data is transmitted from the coupling circuit to a display.

Other objects of the invention and its particular features and advantages will become more apparent from consideration of the following drawings and accompanying detailed description.

### Brief Description Of The Drawings

Figure 1 is a block diagram of one advantageous embodiment of the present invention.

Figure 2 is a block diagram of the video/illumination module according to Figure 1.

Figure 3 is a block diagram of the video system according to Figure 1.

Figure 4 is an illustration of a video laryngoscope with a curved blade according to Figure 1.

Figure 4A is an alternate embodiment according to Figure 4.

Figure 5 is an illustration of the curved blade detached from the handle according to Figure 4.

Figure 5A is an alternate embodiment according to Figure 5.

Figure 6 is an illustration of a video laryngoscope with a straight blade according to Figure 1.

Figure 6A is an alternate embodiment according to Figure 6.

Figure 7 is an illustration of a rigid endoscopic device according to Figure 1.

Figure 7A is an alternate embodiment according to Figure 7.

Figure 8 is an illustration of a flexible endoscopic device according to Figure 1.

Figure 8A is an alternate embodiment according to Figure 8.

Figure 9 is an illustration of another advantageous embodiment of the present invention according to Figures 1, 4 and 7-8.

### Detailed Description Of The Drawings

Referring now to the drawings, wherein like reference numerals designate corresponding structure throughout the views.

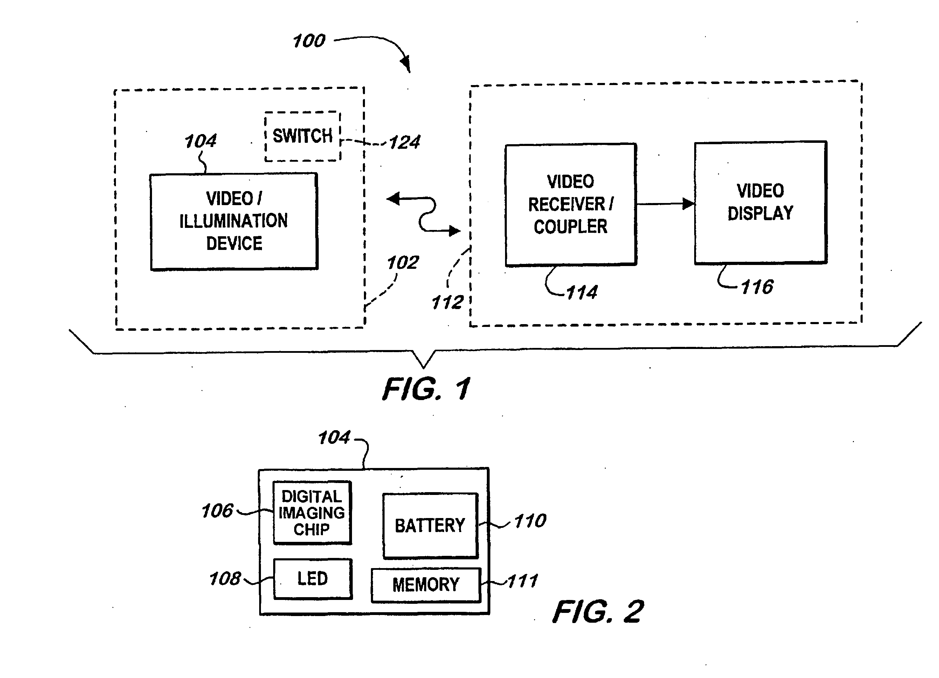

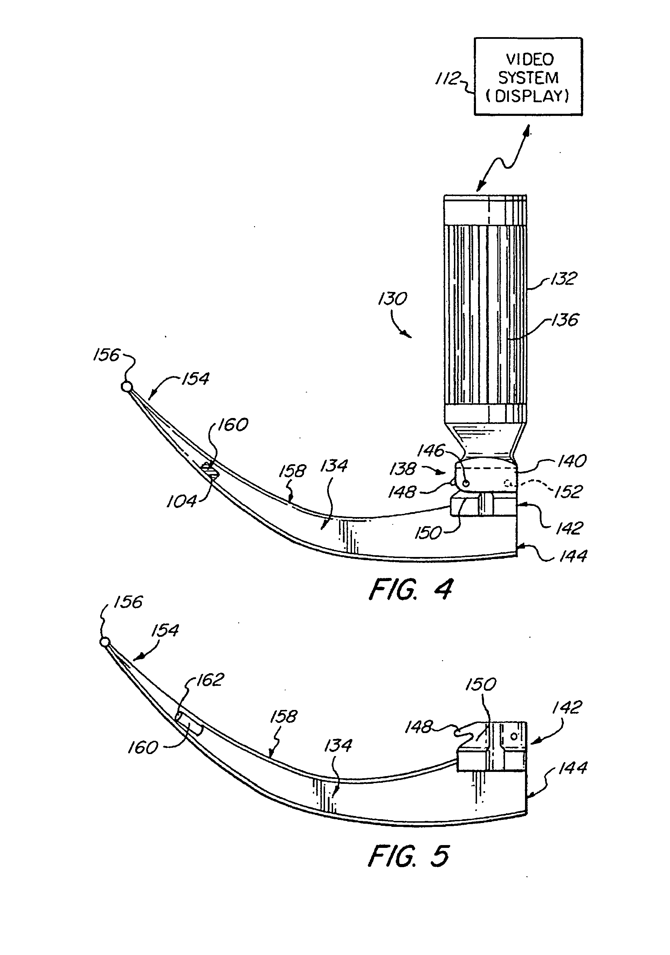

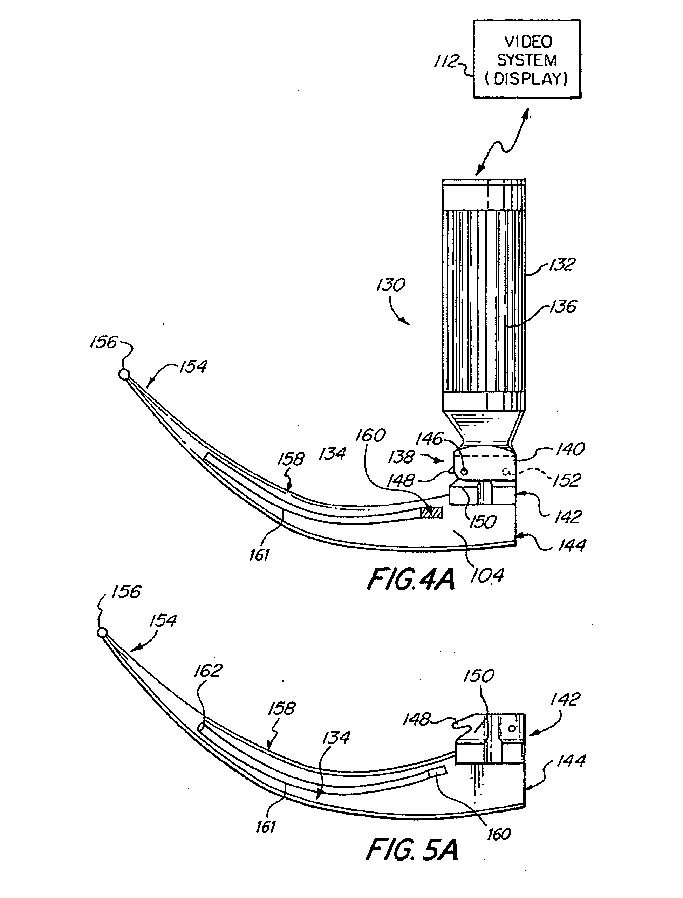

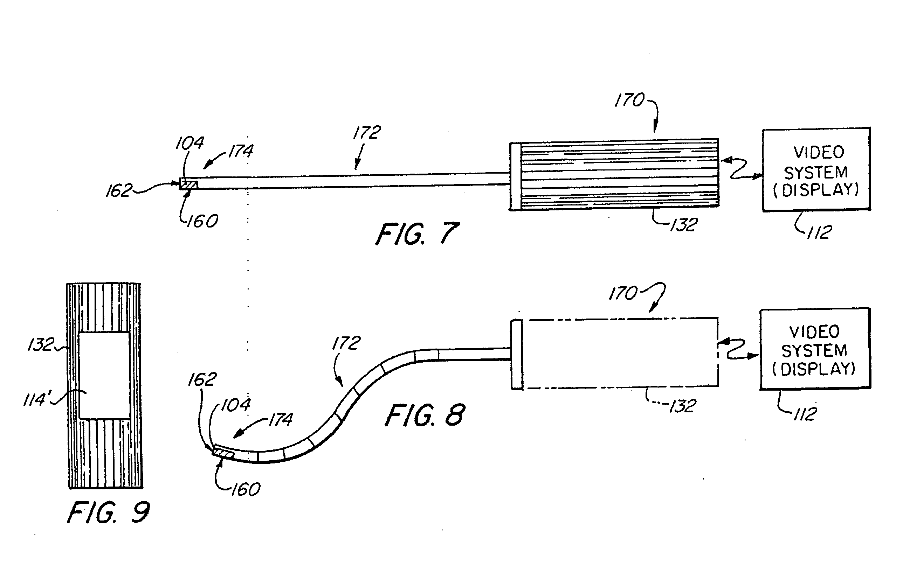

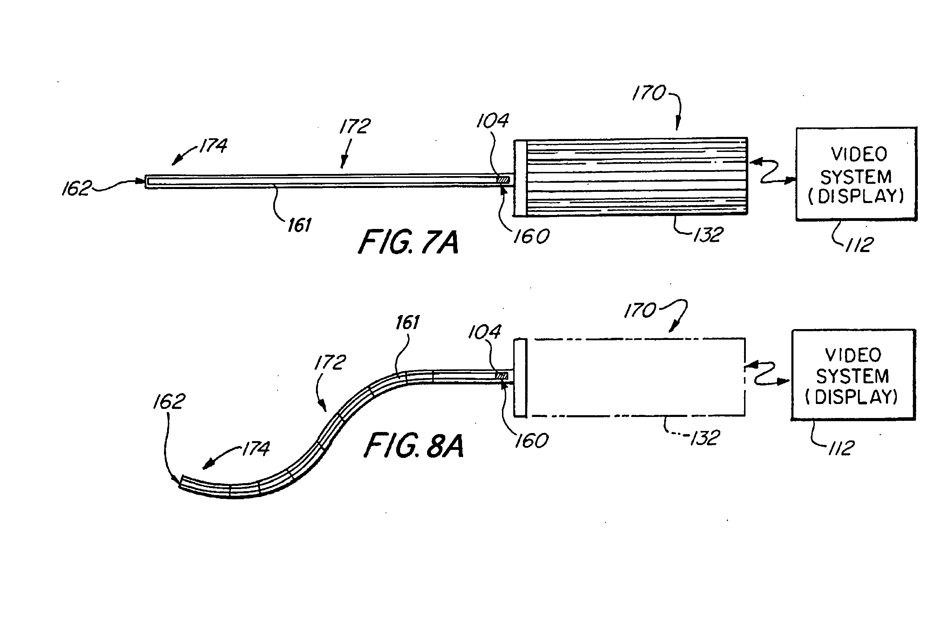

A video system 100 for use with an endoscopic device 102 is depicted in Figure 1. It is contemplated that the endoscopic device 102 may comprise, for example, a laryngoscope 130 as depicted in Figures 4 - 6, or an endoscope 170 as depicted in Figures 7-8.

A video/illumination device 104 is located in endoscopic device 102 and may comprise a digital imaging chip 106, an LED 108, a power source 110 such as a battery, and a memory 111 as illustrated in Figure 2. The LED 108 is very compact in size yet may provide for illumination of an area to be viewed, such as, for example, an area ahead of the endoscopic device 102. The battery 110 may comprise any battery type as is commonly used in industry and is contemplated that it may have a twelve-hour battery life. Further, battery 110 may in one advantageous embodiment be rechargeable.

Referring back to Figure 1, video/illumination device 104 may pick up reflected light from an area to be viewed and translates the reflected light into image data that may be transmitted to video system 112. This transmission may advantageously be wireless. The transmission may comprise any acceptable transmission means including but not limited to for example, radio-frequency transmission.

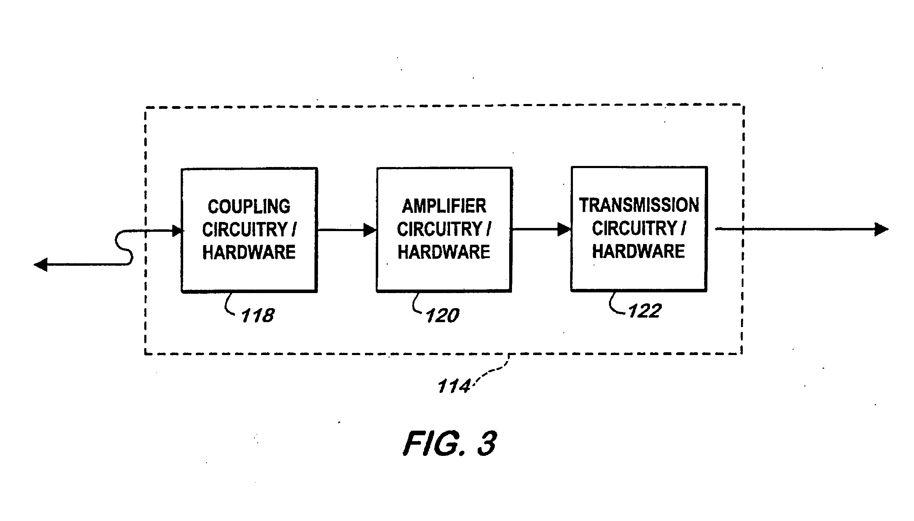

Video system 112 may, in one advantageous embodiment comprise a video receiver/coupler 114 and a video system/display 116. Video receiver/coupler 114 may comprise any type of electronic circuitry and/or hardware for receiving the image data generated by video/illumination device 104. It is contemplated that video receiver/coupler 114 may comprise for example, coupling circuitry or hardware (118), amplification circuitry or hardware (120) and transmission circuitry or hardware (122) as depicted in Figure 3.

The wireless transmission between video/illumination device 104 and video system 112 is illustrated in Figure 1 as a curved line with arrows in two different directions. It is contemplated that upon initiation of video system 100 the video receiver/coupler 114 can "hand-shake" with video/illumination device 104 establishing communication therebetween. In addition, it is contemplated that information relating to the video/illumination device 104 may be downloaded from memory 111 by video receiver/coupler 114 related to for example, configuration data, use data and/or maintenance data. This is especially useful where different video receiver/couplers 114 are used with differing endoscopic devices. The data for example may inform the physician of the total number of hours of use for the particular video receiver/coupler 114 and provide a message relating to scheduled or required maintenance needed. It is further contemplated the data on memory 11 may be updated, especially related to system use and maintenance.

Once video system 112 has identified and established communication with endoscopic device 102, command signals may be sent to video/illumination device 104 to turn LED 108 on. It is contemplated that the command signals may be automatic upon establishment of communication or may advantageously be manual via a switch 124 located on the endoscopic device 102 as seen in Figure 1.

Video system/display 116 may comprise virtually any commercially available video system and monitor for display of the image data generated by video/illumination device 104.

In Figure 4 endoscopic device 102 comprises a video laryngoscope 130, having handle 132 along with the attached blade 134.

The handle 132 is typically cylindrical with a knurled outer surface 136 thereby facilitating a secure gripping surface. As is shown in Figure 5, the handle 132 is detachably joined to a blade 134, which in this instance is curved, by a hinge-type joinder 138. This curved type blade 134 is also known as the well-known Mclntosh blade.

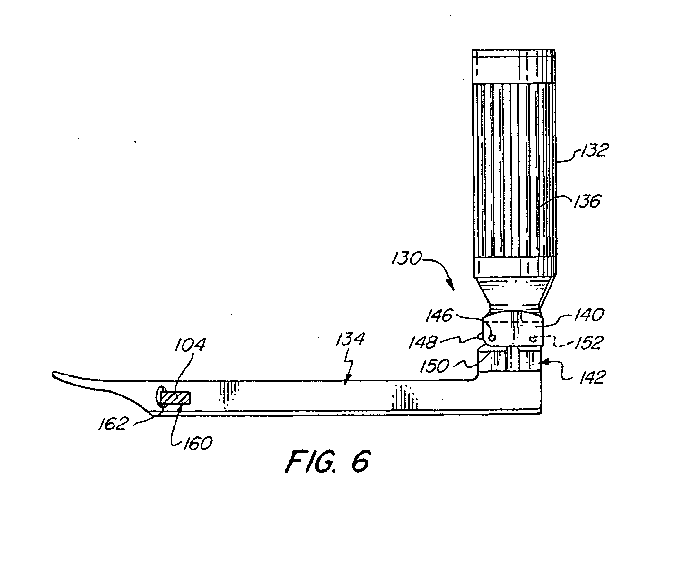

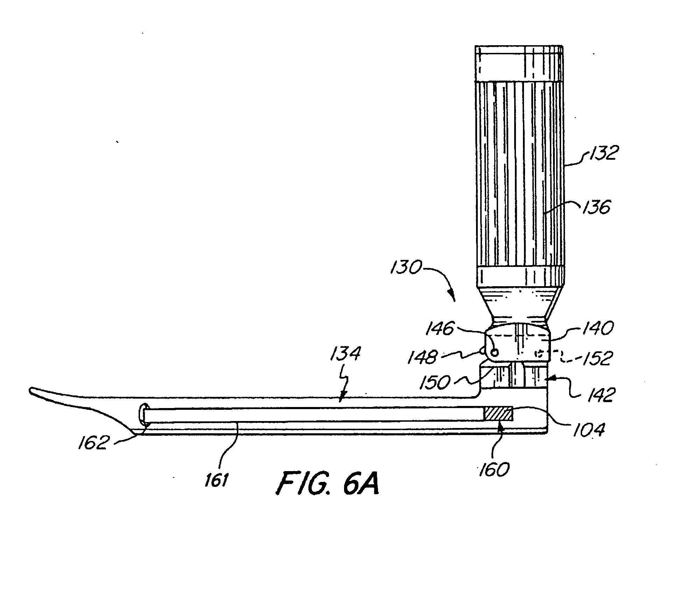

The hinge-type joinder 138 includes a pair of conventional hinge socket 140 and connector 142 respectively mounted to the lower end of the handle 136 and to a proximal end 144 of the blade 134. Socket 140 further includes a crossbar 146. Connector 142 includes a hook 148 in a block 150 that fits into socket 140 as seen in Figures 4 and 5. The hook 148 engages the crossbar 146, and the handle 132 is rotated 90 degrees so that the blade 134 will be rigidly held to the handle 132. This is a common hinge-type joinder 138 used in this type of instrumentation and is useful for all blade forms, of which the two illustrated forms (Figures 4 and 6) are merely examples. A ball detent 152 detachably retains the handle 132 and blade 134 together and erect in the assembled configuration. The assembled instrument is rigid during the procedure.

Blade 132 has a distal end 154 which may be smoothed by a bulb-like edge 156. It has a curved top surface 158 extending from the distal end 154 toward the proximal end 144. This top surface 158 is used to elevate the tongue and permit the visualization of the vocal cords beneath it.

As seen in Figures 4 and 5, blade 134 additionally includes cavity 160 at the distal end 154 of the blade 134. The cavity 160 is designed to receive video/illumination device 104 therein. Cavity 160 may further include in one advantageous embodiment clear window 162, which may act to protect video/illumination device 104. It is further contemplated that video/illumination device 104 may or may not be removable from cavity 160.

As seen in Figure 4, video/illumination device 104 may be positioned in cavity 160 at, for example, at distal end 154 of blade 134 so as to illuminate the area ahead of blade 134. Video/illumination device 104 is further positioned to pick-up reflected light from the area ahead of blade 134, to generate image data corresponding to the reflected light. The image data may then advantageously be wirelessly transmitted to video system 112 for display.

Referring now to Figures 4A and 5A, an alternative embodiment of the present invention is illustrated. In this embodiment, video/illumination device 104 is located at a proximal end of blade 134. While video/illumination device 104 is illustrated as located at the proximal end of blade 134, it is contemplated that, for example, a digital imaging chip 106 and/or an LED 108 may individually or both be positioned at the proximal end. In this embodiment, an illumination/image guide 161 is provided for transmitting the illuminating light generated by LED 108 to the distal end of the blade 134, and for transmitting reflected light back to the digital imaging chip 106. Digital imaging chip 106 may comprise, for example but is not limited to, a CCD or a C-Mos chip. Alternatively, video/illumination device 104 may be positioned in handle 136.

In the case that only LED 108 is positioned at the distal end of blade 134, illumination/image guide 161 need only comprise an image guide for transmitting reflected light back to digital imaging chip 106. Likewise, in the case that only digital imaging chip 106 is positioned at the distal end of blade 134, illumination/image guide 161 need only comprise an illumination guide for transmitting illuminating light to the area to be viewed.

Referring now to Figure 9 an alternative embodiment of the present invention is shown. Video receiver/coupler 114' is positioned, for example, in handle 132. In this advantageous embodiment, video/illumination device 104 located at the distal end 154 of blade 134, wirelessly sends image data to video receiver/coupler 114' such that no cabling or optical guides are required to extend between the distal end 154 of the blade 134 and the handle 132. The transmitted image data received by video receiver/coupler 114' may then be retransmitted to video receiver/coupler 114 for display to the user. It is contemplated that the transmission of image data from video receiver/coupler 114' to video receiver/coupler 114 may be a wireless transmission. Alternatively, an optical cable may be provided extending from laryngoscope 130 to video system 112. In any event, the transmission of image data from video/illumination device 104 is provided as a wireless transmission.

Turning now to Figure 6, an alternative configuration of video laryngoscope 130 is provided. In this configuration, video laryngoscope 130 is similar to that described in connection with Figures 4 and 5, but is provided with a straight blade 134. This is the well-known Foregger-Magill blade. It is contemplated that the invention may equally be used with many differing configurations, and that the particular configurations illustrated in Figures 4 - 6 are provided merely as examples and not provided as a limitation. It will be evident to the physician that the invention may be used with virtually any laryngoscope configuration, which is selected by the physician according to the needs of the patient.

It is further contemplated that the invention may equally have application in neo-natal intubation procedures in which the diameter of the laryngoscope is very small due to anatomical structures of infants and premature babies. These types of extremely small diameter laryngoscopes are typically flexible for at least a portion of the insertion section. A wireless transmission system provides significant advantages therefore because the insertion portion does not need to contain either an illumination or an image guide.

Referring to Figure 6A, and alternative embodiment to Figure 6 is illustrated with video/illumination device 104 positioned at a proximal end of blade 134. This advantageous embodiment is similar to the embodiment described in connection with Figures 4A and 5A and therefore will not be redescribed here.

Turning now to Figures 7 and 8, an endoscope 170 is illustrated as endoscopic device 102. It is contemplated that endoscope 170 may comprise a handle 132, as previously discussed in connection with figures 4 - 6, and a shaft 172. The shaft 172 may comprise a rigid member as illustrated in Figure 7, or may advantageously comprise a flexible member for at least a portion of the shaft 172, as illustrated in Figure 8. The endoscope shaft 170, whether rigid or flexible may be attached to handle 132 via any well known connection mechanism in the art.

A cavity 160 is located at a distal end 174 of shaft 170. Cavity 160, as previously discussed, is provided to receive video/illumination device 104 therein. Additionally, in one advantageous embodiment, a window 162 is provided on cavity 160 to for example, enclose and protect video/illumination device 104.

It is further contemplated that video receiver/coupler 114' may be positioned in handle 132 as illustrated in Figure 9 and may operate in a manner as previously discussed. Additionally, endoscope 170 may be provided with a wireless connection to video system 112, or may be provided with cabling (not shown) to couple endoscope 170 to video system 112.

Figures 7A and 8A illustrate alternative embodiments to those illustrated in Figures 7 and 8, with video/illumination device 104 positioned at a proximal end of shaft 172. Again, it is contemplated that either digital imaging chip 106 and/or LED 108 may be positioned at the proximal end of shaft 172. Alternatively, digital imaging chip 106 may be positioned at the distal end while LED 108 is positioned at the proximal end or vice versa. In any event, it is contemplated that if either digital imaging chip 106 or LED 108 or both are located at the proximal end of shaft 172, illumination/image guide 161 is provided for transmitting the illuminating light to and reflected light from the area to be viewed as described in connection with Figures 4A and 5A. Alternatively, imaging chip 106 and/or LED 108 may be positioned in handle 132.

While the present invention has been described in connection with a video laryngoscope and a video endoscope, these are merely two applications in which the invention may be utilized and are not intended to exhaust all possible applications. Rather, it is contemplated that the present invention may effectively be utilized in many varying application in which an image picked up by a digital imaging chip is wirelessly transmitted for display to a user.

Although the invention has been described with reference to a particular arrangement of parts, features and the like, these are not intended to exhaust all possible arrangements or features, and indeed many other modifications and variations will be ascertainable to those of skill in the art.

## Claims

1. A video endoscope system for displaying image data to a user comprising:
an endoscopic device for coupling to a video system, said endoscopic device having a proximal end connected to a handle and a distal end and including:
a video/illumination device associated with said endoscopic device, said video/illumination device having an illumination device and a battery for illuminating an area to be viewed, and a digital imaging chip for picking up reflected light from the area and generating image data; and
said image data wirelessly transmitted to said video system for display to a user.

2. The video endoscope system according to Claim 1 wherein said video/illumination device is positioned at the distal end of said endoscopic device.

3. The video endoscope system according to Claim 1 wherein said endoscopic device comprises an endoscope.

4. The video endoscope system according to Claim 3 wherein said endoscope comprises a flexible endoscope.

5. The video endoscope system according to Claim 1 wherein said endoscopic device comprises a laryngoscope.

6. The video endoscope system according to Claim 1 wherein said video/illumination device is located in a cavity in said endoscopic device.

7. The video endoscope system according to Claim 6 wherein said cavity further includes a window.

8. The video endoscope system according to Claim 1 wherein said video/illumination device is removable from said endoscopic device.

9. The video endoscope system according to Claim 1 wherein said illumination device comprises an LED.

10. The video endoscope system according to Claim 1 wherein said video system comprises a video display.

11. The video endoscope system according to Claim 1 further comprising a coupler positioned in the handle of said endoscope for receiving said image data.

12. The video endoscope system according to Claim 11 further comprising a video display wirelessly coupled to said coupler for displaying the image data to the user.

13. The video endoscope system according to Claim 1 further comprising a coupler located remotely from said endoscope for receiving said image data.

14. A method for viewing an area with an endoscopic device comprising the steps of:
positioning a digital imaging chip and an illuminating device on the endoscopic device;
wirelessly coupling the endoscopic device to a video system;
illuminating an area to be viewed with the illumination device and powered by a battery;
generating image data based on reflected light picked up by the digital imaging chip;
wirelessly transmitting the image data to the video system; and
displaying the image data to a user.
